# EUROPEAN PATENT SPECIFICATION

(11) **EP 3 761 024 B1**
(45) Date of publication and mention of the grant of the patent: **05.06.2024**
(21) Application number: 18907696.1
(22) Date of filing: 17.12.2018
(51) Int. Cl.: G01N 30/88, A23D 9/02, C11C 3/10, G01N 30/00, G01N 30/06, G01N 33/03, C11C 1/02, C11C 3/00, C11B 3/00, A23D 9/007, G01N 30/14

(54) **METHOD FOR QUANTIFYING DIALKYL KETONE IN OIL AND FAT**
VERFAHREN ZUR QUANTIFIZIERUNG VON DIALKYLKETON IN ÖL UND FETT
PROCÉDÉ DE QUANTIFICATION DE DIALKYLCÉTONE DANS DE L'HUILE ET DE LA GRAISSE

(30) Priority: 28.02.2018 JP 2018034480
(43) Date of publication of application: 06.01.2021
(73) Proprietor: The Nisshin OilliO Group, Ltd., Tokyo 104-8285 (JP)
(72) Inventor: ABE, Kosuke, Yokohama-shi, Kanagawa 235-8558 (JP); ITO, Mineko, Yokohama-shi, Kanagawa 235-8558 (JP); SATANI, Tae, Yokohama-shi, Kanagawa 235-8558 (JP); YANAGIHASHI, Akiko, Yokohama-shi, Kanagawa 235-8558 (JP); MURANO, Yoshihiro, Yokohama-shi, Kanagawa 235-8558 (JP)
(74) Representative: Winter, Brandl - Partnerschaft mbB
(86) International application number: PCT/JP2018/046394
(87) International publication number: WO 2019/167390

(56) References cited:
- JP-A- H06 160 379
- JP-A- 2003 322 594
- JP-A- 2008 224 563
- JP-A- 2012 224 797
- JP-A- 2013 153 742
- JP-A- 2016 003 923
- US-A- 5 504 231
- GHAZANI SAEED MIRZAEE ET AL: "Micronutrient content of cold-pressed, hot-pressed, solvent extracted and RBD canola oil: Implications for nutrition and quality", EUROPEAN JOURNAL OF LIPID SCIENCE TECHNOLOGY, vol. 116, no. 4, 20 January 2014 (2014-01-20), pages 380-387, XP055862640, DE ISSN: 1438-7697, DOI: 10.1002/ejlt.201300288
- ONTANON, I. et al.: "A modified commercial gas chromatograph for the continuous monitoring of the thermal degradation of sunflower oil and off-line solid phase extraction gas-chromatography-mass spectrometry characterization of released volatiles", Journal of Chromatography A, vol. 1388, 2015, pages 52-59, XP029145025, DOI: doi:10.1016/j.chroma.2015.02.001

## Description

### Technical Field

The present invention relates to a method of quantifying dialkyl ketones in an oil and/or fat.

### Related Background Art

One of methods of reforming an oil and/or fat (edible oil in particular) is a transesterification method. In a chemical transesterification method using an alkali as a catalyst, dialkyl ketones (hereinafter also referred to as "DAKs") are produced as by-products as well as a reformed oil and/or fat. When the amount of DAKs is large, they are removed by molecular distillation or the like.

Also, a method of producing an oil and/or fat with reduced DAKs has been known from JP 2012-224797 A and US 5 504 231 A.

Performing a solid-phase extraction before a gas chromatograph has been known e.g., form Ghazani Saeed Mirzaee et al ("Micronutrient content of cold-pressed, hotpressed,solvent extracted and RBD canola oil: Implications for nutrition and quality", European Journal of Lipid Science Technology, vol. 116, no. 4, 20 January 2014 (2014-01-20), pages 380-387).

### Summary of Invention

### Problem to be solved by the invention

It is necessary to accurately quantify the DAKs in an oil and/or fat for the determination of the conditions for the transesterification reaction, the determination of whether it is necessary to remove the DAKs after the transesterification reaction, and the development of a new DAKs removal method.

However, in the case of quantitative analysis by testing a transesterification reaction product as it is with a gas chromatograph (GC), the present inventors have found for the first time that phytosterols contained in the reaction product behave similarly to the DAKs in the GC and prevent accurate quantification of the DAKs.

### Means for solution of the problem

To solve the above-described problem, the present inventors have conducted intensive study. As a result, the present inventors have found that use of a product of solid-phase extraction of an oil and/or fat in a GC enables accurate identification and quantification of the DAKs. This finding had led to the completion of the present invention. Specifically, the present invention relates to the method of quantifying dialkyl ketones in an oil and/or fat as specified in claim 1. The present invention further relates to the method of producing an oil and/or fat by a transesterification reaction using an alkali catalyst as specified in claim 3.

### Effect of Invention

As shown by the examples to be described later, according to the method of the present invention, it is possible to accurately quantify the DAKs in an oil and/or fat (in particular, a product of a transesterification reaction of an oil and/or fat.

### Description of Embodiments

An "oil and/or fat" includes, but not particularly limited to, for example, vegetable oils and/or fats and animal oils and/or fats, but vegetable oils and/or fats are preferable.

The vegetable oils and/or fats include canola oil, palm oil, etc., and transesterified oils of these, and transesterified oils are preferable.

Also, edible oils and/or fats are preferable oils and/or fats.

The present invention is preferably applicable to oils and/or fats each being the "product of a transesterification reaction of an oil and/or fat using an alkali catalyst".

As the "transesterification reaction of an oil and/or fat using an alkali catalyst", any of those used in the field of oil and/or fat production can be used without any particular limitation.

The alkali catalyst includes sodium hydroxide, potassium hydroxide, sodium carbonate, potassium carbonate, and the like.

As the condition for the transesterification reaction, any of those commonly used for a transesterification reaction of an oil and/or fat can be employed without any particular limitation.

### <Method of Quantifying Dialkyl Ketones in Oil and/or Fat>

A method of quantifying dialkyl ketones in an oil and/or fat according to the present invention includes the steps of: performing solid-phase extraction on the oil and/or fat; and quantifying the product of the solid-phase extraction with a gas chromatograph or a liquid chromatograph.

As the "solid-phase extraction", any of those used for oils and/or fats can be used without any particular limitation. The purpose of the "solid-phase extraction" is to separate and remove phytosterols that prevent the subsequent quantification with the gas chromatograph or liquid chromatograph from being performed accurately. Therefore, a solid-phase extraction method capable of efficiently separating and removing the phytosterols and the like is used in the present invention.

Here, the "solid-phase extraction" is also called SPE and refers to an extraction method in which, by using the affinity between a target substance (solute) contained in a sample and a carrier (stationary phase) or the like, the solute is adsorbed onto the support or eluted as is to separate the solute. In the present invention, the DAKs contained in an oil and/or fat are adsorbed onto a support, and then the adsorbed DAKs are extracted with a solvent to be separated from the phytosterols.

As for the "support" used in the solid-phase extraction, the stationary phase includes an aminopropyl group, and silica gel or graphite carbon.

The "solvent" used in the solid-phase extraction includes hexane, chloroform, and the like, and hexane and chloroform are preferable. A single kind of solvent may be used alone, or two or more kinds of solvents may be combined as appropriate.

As the conditions for the solid-phase extraction, any conditions used for extraction of oils and/or fats can be employed without any particular limitation.

Prior to the solid-phase extraction, the oil and/or fat is saponified, and further the saponified product is subjected to liquid-phase extraction, since doing so can make the quantifiability of the DAKs higher.

As the conditions for saponification, any conditions used for saponification of oils and/or fats can be employed without any particular limitation. For the saponification, it is possible to use an alkaline aqueous solution or an alkaline alcohol solution, for example, and it is preferable to use an alcohol solution of sodium hydroxide, potassium hydroxide, or the like, for example. Methanol, ethanol, propanol, butanol, or the like can be used as the alcohol. The alcohol solution may contain water. The saponification temperature is preferably 30 to 120°C and more preferably 70 to 100°C.

In order to remove the water-soluble component produced in the saponification of the oil and/or fat, liquid-phase extraction is performed. For example, it is possible to extract the saponified product with an organic solvent with low solubility to water. Ether, hexane, heptane, octane, petroleum ether, benzene, toluene, xylene, dichloromethane, or the like can be used as the organic solvent. Considering the efficiency of the concentrating operation (solvent removal) to be performed after this extraction, an organic solvent with a boiling point of 100°C or less is preferable. After the extraction operation, a washing operation (e.g., water washing) is preferably performed in order to remove the alkali in the organic phase.

After performing the saponification, the liquid-phase extraction, and the washing, a concentrating operation is preferably performed to raise the concentration of the DAKs. The concentrating method includes, but not particularly limited to, distilling the organic solvent after drying with a desiccant such as anhydrous sodium sulfate, calcium chloride, or a molecular sieve.

The "gas chromatograph (GC)" only needs to be applicable to oils and/or fats, and a capillary GC having a FID detector or a mass spectrometer can be used without any particular limitation. The kind of the filler in the capillary column for measurement is preferably, but not particularly limited to, dimethyl polysiloxane, diphenyl dimethyl polysiloxane, or the like.

As the conditions for the GC, any conditions used for analysis of oils and/or fats can be employed without any particular limitation.

The "liquid chromatograph (LC)" only needs to be applicable to oils and/or fats, and one having an evaporative light scattering detector, a differential refractometer, a mass spectrometer, or the like can be used without any particular limitation. The kind of the stationary phase in the measurement column is preferably, but not particularly limited to, a normal-phase column with silica gel or the like or a reversed-phase column with C18 or the like.

As the conditions for the LC, any conditions used for analysis of oils and/or fats can be employed without any particular limitation.

In the present invention, many kinds of dialkyl ketones (DAKs) contained in an oil and/or fat can be quantified.

The DAKs include compounds represented by a general formula (1): R₁C(O)R₂ (in the formula, R₁ and R₂ each independently represent an alkyl group with C₁ to C₂₄). Specific examples include 9-heptadecanone (a compound with R₁ and R₂ in the general formula (1) being alkyl groups with Cs), 10-nonadecanone, 11-heneicosanone, 14-heptacosanone, 16-hentriacontanone, 18-pentatriacontanone, and 12-tricosanone. However, other DAKs (e.g., 9,26-pentatriacontadien-18-one) are quantifiable as well.

### <Method of Producing Oil and/or FAT by Transesterification Reaction>

It is possible to quantify the DAKs in the product of a transesterification reaction by following the above <Method of Quantifying Dialkyl Ketones in Oil and/or Fat>, and determine whether it is necessary to remove the DAKs from the product based on the result of the quantification.

Thus, the present invention includes a method of producing an oil and/or fat by a transesterification reaction, comprising the step of: quantifying the dialkyl ketones in the oil and/or fat after the transesterification reaction by following the method described in the above <Method of Quantifying Dialkyl Ketones in Oil and/or Fat>; wherein the condition for refining and/or blending after the quantification step is determined based on the amount of the quantified dialkyl ketones.

In the present invention, the quantification step only needs to be after the transesterification, and may be before the refining step and/or the blending step or in the middle of the refining step and/or the blending step.

The step of refining the transesterified oil includes at least one of neutralization of the alkali catalyst with an acidic substance or washing with water, deacidification, decolorization, deodorization, fractionation, and the like, and one may be before or after or in the middle of another.

For example, when it is found in the quantification step that the amount of the DAKs in the transesterified oil is large, the amounts of the DAKs can be reduced by additionally performing a washing step with an acidic liquid (e.g., citric acid). In the case of performing washing with an acidic liquid, it is preferable to perform water washing and deodorization after that. The DAKs can alternatively be removed by additionally performing a distillation step under high vacuum, such as short-path distillation.

When it is found in the quantification step that the amount of the DAKs in the transesterified oil is small (or zero), it is possible to choose not to perform these refining steps.

In the case of blending a plurality of transesterified oils, it is possible to combine a lot with a large amount of DAKs and a lot with a small amount of the DAKs to produce a blend oil whose amount of the DAKs is kept within a certain standard. Thus, the DAKs quantification method of the present invention can also be used to determine the condition for the blending in blend oil production. In particular, when a plurality of product tanks store transesterified oils with different amounts of DAKs, it is preferable to quantify the amount of the DAKs in a newly produced transesterified oil before or in the middle of its refining and introduce the transesterified oil after the refining into an appropriate product tank, since doing so can adjust the amount of the DAKs in the transesterified oil in each product tank (e.g., keep the amount of the DAKs low).

### [Examples]

Hereinafter, the present invention will be described further in detail based on examples. However, the present invention is not limited to these.

### [Example 1]: Quantitative Analysis of DAKs in Oil and/or Fat (1)

### (Sample Preparation)

The quantification method of the present invention was evaluated using oil and/or fat samples prepared in advance so as to contain predetermined amounts of DAKs. Specifically, samples 1 to 5 were used which were prepared by adding respective DAKs standard reagents to canola oil containing no DAKs (produced by The Nisshin OilliO Group, Ltd.) in the blending amounts in table 1.

**[Table 1]**

| | DAKs | Blending amount of DAKs (ppm (in terms of mass) |
|---|---|---|
| Sample 1 | 9-heptadecanone | 60.0 |
| Sample 2 | 10-nonadecanone | 60.5 |
| Sample 3 | 14-heptacosanone | 79.5 |
| Sample 4 | 16-hentriacontanone | 74.5 |
| Sample 5 | 18-pentatriacontanone | 52.0 |

Before the solid-phase extraction, the following saponification operation and extraction operation (washing operation and a dehydration-drying operation) were performed on each sample to produce an extract.

### (Saponification Operation)

1g of the sample and 10 mL of 2N potassium hydroxide/ethanol were added into a recovery flask and saponificated by refluxing for 30 minutes at 90°C. Thereafter, the reactant was cooled to room temperature (20°C), followed by addition of 20 mL of water and mixing, and transfer to a separatory funnel. Then, 30 mL of petroleum ether was added, followed by shaking, and the upper layer (petroleum ether layer) was collected (first time). 30 mL of petroleum ether was added to the remaining lower layer, followed by shaking, and the upper layer (petroleum ether layer) was collected (second time) and mixed with the upper layer collected in the first time. Further, 30 mL of petroleum ether was added to the remaining lower layer, followed by shaking, and the upper layer (petroleum ether layer) was collected (third time) and mixed with the upper layers collected in the first and second times.

### (Washing Operation)

40 mL of water/ethanol (1:1 v/v) was added to the upper layer (petroleum ether layer) obtained by the above saponification operation, followed by shaking, and the upper layer (petroleum ether layer) was collected. 40 mL of water/ethanol (1:1 v/v) was added to the above upper layer, followed by shaking, and the upper layer (petroleum ether layer) was collected. Further, 40 mL of water/ethanol (1:1 v/v) was added to the above upper layer, followed by shaking, and the upper layer (petroleum ether layer) was collected.

### (Dehydration-Drying operation)

10 g of anhydrous sodium sulfate was added to the upper layer (petroleum ether layer) collected in the above washing operation, and the mixture was shaken to be mixed to thereby dehydrate the upper layer. The dehydrated upper layer (petroleum ether layer) was filtered through filter paper to remove the anhydrous sodium sulfate, and further the solvent was removed with a rotary evaporator. As a result, an extract was obtained.

### (Solid-Phase Extraction Operation)

The following solid-phase extraction (SPE) was performed on the extract obtained by the above operations to obtain a dialkyl ketone-containing extract (fraction) with phytosterols removed.

0.5 mL of hexane/chloroform (9:1 v/v) was added into a commercially available solid-phase extraction tube (Supelclean ENVI-Carb/NH₂ (by Sigma-Aldrich), a stationary phase of an aminopropyl group and graphite carbon), and the extract was loaded. Then, 15 mL of hexane/chloroform (9:1 v/v) was added into the solid-phase extraction tube, the eluted substance was collected, and the solvent was removed with the rotary evaporator to thereby obtain a fraction 1.

Then, 20 mL of hexane/chloroform (7:3 v/v) was added into the solid-phase extraction tube, the eluted substance was collected, and the solvent was removed with the rotary evaporator to thereby obtain a fraction 2.

Then, 10 mL of chloroform was added into the solid-phase extraction tube, the eluted substance was collected, and the solvent was removed with the rotary evaporator to thereby obtain a fraction 3.

Then, 50 mL of chloroform was added into the solid-phase extraction tube, the eluted substance was collected, and the solvent was removed with the rotary evaporator to thereby obtain a fraction 4.

### (Gas Chromatograph Operation)

0.5 mL of chloroform was added to each fraction, and the dissolved product was set in a gas chromatograph under the following conditions.

Note that chloroform solutions of the above-mentioned dialkyl ketone standard substances were used as standard samples (blending amount: 50 ppm (in term of mass)). Gas Chromatograph Conditions
Capillary GC apparatus having an FID detector: GC 7890B manufactured by Agilent Technologies, Inc.
Column: DB1-HT manufactured by Agilent Technologies, Inc., length 15 m × inner diameter 0.32 mm × thickness 0.1 µm (filler: 100% dimethylpolysiloxane)
Carrier gas: helium
Inlet temperature: 330°C
Detector temperature: 340°C
Oven temperature: 150°C to 340°C (temperature increase: 20°C/min)
Injection volume: 1 µL
Split ratio: 50:1
Flow rate: 2 mL/min
Standard substance: dialkyl ketone reagent

The samples' peaks and the standard samples' peaks were compared to calculate the amounts of the DAKs. Table 2 shows the result. The recovery rates in table 2 are values based on the assumption that the amounts (in terms of mass) of the DAKs in the samples before being subjected to the quantification method are 100%.

**[Table 2]**

| | Amount of DAKs (ppm (in terms of mass) | Recovery rate (%) |
|---|---|---|
| Sample 1 | 52.4 (9-heptadecanone) | 87 |
| Sample 2 | 57.0 (10-nonadecanone) | 94 |
| Sample 3 | 77.6 (14-heptacosanone) | 98 |
| Sample 4 | 57.5 (16-hentriacontanone) | 77 |
| Sample 5 | 43.1 (18-pentatriacontanone) | 83 |

With the above-described quantification method, the DAKs were detected at a recovery rate as high as 77 to 98% relative to the amounts of the DAKs in the samples before the quantification.

This result indicates that the method of the present invention is capable of accurately quantifying the DAKs in an oil and/or fat.

### [Example 2]: Quantitative Analysis of DAKs in Oil and/or Fat (2)

### (Sample Preparation)

The quantification method of the present invention was evaluated using oil and/or fat samples prepared in advance so as to contain predetermined amounts of DAKs. Specifically, samples 6 to 10 were used which were prepared by adding respective DAKs standard reagents to palm oil containing no DAKs (produced by The Nisshin OilliO Group, Ltd.) in the blending amounts in table 3.

**[Table 3]**

| | DAKs | Blending amount of DAKs (ppm (in terms of mass) |
|---|---|---|
| Sample 6 | 10-nonadecanone | 61.1 |
| Sample 7 | 12-tricosanone | 50.9 |
| Sample 8 | 14-heptacosanone | 53.1 |
| Sample 9 | 18-pentatriacontanone | 47.6 |
| Sample 10 | 9,26-pentatriacontadien-18-one | 57.6 |

Before the solid-phase extraction, a saponification operation and an extraction operation (washing operation and a dehydration-drying operation) similar to those in example 1 described above were performed on each sample to produce an extract.

The solid-phase extraction operation and the gas chromatograph operation were also similar to those in example 1 described above.

The samples' peaks and the standard samples' peaks were compared to calculate the amounts of the DAKs. Table 4 shows the result. The recovery rates in table 4 are values based on the assumption that the amounts (in terms of mass) of the DAKs in the samples before being subjected to the quantification method are 100%.

**[Table 4]**

| | Amount of DAKs (ppm (in terms of mass) | Recovery rate (%) |
|---|---|---|
| Sample 6 | 49.8 (10-nonadecanone) | 82 |
| Sample 7 | 42.4 (12-tricosanone) | 83 |
| Sample 8 | 44.7 (14-heptacosanone) | 84 |
| Sample 9 | 45.7 (18-pentatriacontanone) | 96 |
| Sample 10 | 47.4 (9,26-pentatriacontadien-18-one) | 82 |

With the above-described quantification method, the DAKs were detected at a recovery rate as high as 82 to 96% relative to the amounts of the DAKs in the samples before the quantification.

This result indicates that the method of the present invention is capable of accurately quantifying the DAKs in an oil and/or fat.

### Industrial Applicability

The present invention is applicable to the field of oils and/or fats.

## Claims

1. A method of quantifying dialkyl ketones in an oil and/or fat, comprising the steps of:
(A) saponifying the oil and/or fat and performing liquid-phase extraction on the saponified product to produce an extract;
(B) performing solid-phase extraction on the extract to obtain a dialkyl ketone-containing extract with phytosterols removed; and
(C) quantifying the dialkyl ketone-containing extract with a gas chromatograph or a liquid chromatograph.

2. The quantification method according to claim 1, wherein the oil and/or fat to be subjected to the step (A) is a product of a transesterification reaction of the oil and/or fat using an alkali catalyst.

3. A method of producing an oil and/or fat by a transesterification reaction using an alkali catalyst, comprising the step of:
quantifying dialkyl ketones in the oil and/or fat after the transesterification reaction by following the method according to claim 1; and
wherein a condition for refining and/or blending after the quantification step is determined based on an amount of the quantified dialkyl ketones.

## Patentansprüche

1. Verfahren zum Quantifizieren von Dialkylketonen in einem Öl und/oder Fett, umfassend die Schritte:
(A) Verseifen des Öls und/oder Fetts und Durchführen einer Flüssigphasenextraktion mit dem verseiften Produkt, um ein Extrakt herzustellen;
(B) Durchführen einer Festphasenextraktion mit dem Extrakt, um ein dialkylketonhaltiges Extrakt zu erhalten, bei dem die Phytosterine entfernt sind; und
(C) Quantifizieren des dialkylketonhaltigen Extrakts mit einem Gaschromatographen oder einem Flüssigkeitschromatographen.

2. Quantifizierungsverfahren gemäß Anspruch 1, wobei das Öl und/oder Fett, das dem Schritt (A) unterzogen werden soll, ein Produkt einer Umesterungsreaktion des Öls und/oder Fetts unter Verwendung eines Alkalikatalysators ist.

3. Verfahren zum Herstellen eines Öls und/oder Fetts durch eine Umesterungsreaktion unter Verwendung eines alkalischen Katalysators, umfassend die Schritte:
Quantifizieren von Dialkylketonen in dem Öl und/oder Fett nach der Umesterungsreaktion gemäß dem Verfahren nach Anspruch 1; und
wobei eine Bedingung zum Raffinieren und/oder Mischen nach dem Quantifizierungsschritt auf der Grundlage einer Menge der quantifizierten Dialkylketone bestimmt wird.

## Revendications

1. Procédé de quantification de dialkylcétones dans une huile et/ou une graisse, comprenant les étapes de :
(A) saponification de l'huile et/ou de la graisse et réalisation d'une extraction en phase liquide sur le produit saponifié pour produire un extrait ;
(B) réalisation d'une extraction en phase solide sur l'extrait pour obtenir un extrait contenant de la dialkylcétone sans phytostérols ; et
(C) quantification de l'extrait contenant de la dialkylcétone avec un chromatographe en phase gazeuse ou un chromatographe en phase liquide.

2. Procédé de quantification selon la revendication 1, dans lequel l'huile et/ou la graisse devant être soumises à l'étape (A) sont un produit d'une réaction de transestérification de l'huile et/ou de la graisse à l'aide d'un catalyseur alcalin.

3. Procédé de production d'une huile et/ou d'une graisse par une réaction de transestérification à l'aide d'un catalyseur alcalin, comprenant l'étape de :
quantification de dialkylcétones dans l'huile et/ou la graisse après la réaction de transestérification en suivant le procédé selon la revendication 1 ; et
dans lequel une condition de raffinage et/ou de mélange après l'étape de quantification est déterminée sur la base d'une quantité de dialkylcétones quantifiées.
